# EUROPÄISCHE PATENTANMELDUNG

(11) **EP 2 361 606 A2**
(43) Veröffentlichungstag der Anmeldung: **31.08.2011**
(21) Anmeldenummer: 10187413.9
(22) Anmeldetag: 13.10.2010
(51) Int. Cl.: A61K 8/44, A61Q 11/00, A61K 8/19, A61K 8/27, A61K 8/37

(54) **Mund- und Zahnpflege- und -Reinigungsmittel mit Ethyllaurolarginat**

(30) Priorität: 22.02.2010 DE 102010002195
(71) Anmelder: Henkel AG & Co. KGaA, 40589 Düsseldorf (DE)
(72) Erfinder: Adomat, Christel, 40591, Düsseldorf (DE); Barth, Adolf Peter, 42781, Haan (DE); Leinen, Hans-Theo, 40699, Erkrath (DE)

(57) **Zusammenfassung**

Mund- und Zahnpflege- und -reinigungsmittel mit besonders hoher antibakterieller und plaquereduzierender Wirkung ohne geschmackliche Beeinträchtigungen enthalten bezogen auf ihr Gewicht in einem oral akzeptablen Träger 0,01 bis 2,5 Gew.-% Ethyllauroylarginat und 0,00001 bis 2,5 Gew.-% mindestens eines antibakteriellen Mittels aus der Gruppe der Parabene und/oder der Silbersalze und/oder der Kupfersalze und/oder der Zinksalze.

## Beschreibung

Die Erfindung betrifft Zubereitungen zur Mund- und Zahnpflege- und -reinigung, die aufgrund von speziellen Inhaltsstoffen gut gegen den Befall durch Mikroorganismen geschützt sind und eine ausgezeichnete plaquereduzierende Wirkung besitzen, ohne daß es zu Geschmacksbeeinträchtigungen kommt.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern. Es hat nicht an Versuchen gefehlt, Mundgeruch und entzündliche Zustände in der Mundhöhle zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung. Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können. In Einzelfällen kann die Bekämpfung der Bakterien nicht ausreichend sein, um einen Rückgang des entzündlichen Zustandes zu erreichen.

Besonders gut für die orale Anwendung geeignete antibakterielle Mittel sind Cetylpyridiniumchlorid und Chlorhexidin. Alkypyridiniumchloride und Chlorophenylbiguanide besitzen aber den Nachteil, in oralen Anwendungszubereitungen oft nicht stabil einarbeitbar zu sein oder zu Geschmacksbeeinträchtigungen zu führen.

Um die Einsatzmenge herkömmlicher antibakterieller Mittel zu verringern, werden oftmals synergistische Kombinationen mehrerer antibakterieller Mittel eingesetzt. So schlägt die WO 03/043 593 A1 vor, herkömmliche antibakterielle Substanzen wie Triclosan, Phenoxyethanol oder Hexetidin mit Ethyllauroylarginat zu kombinieren, um die antibakterielle Wirkung zu verstärken.

Der Einsatz von Ethyllauroylarginat in Zahnpasten oder als Agens gegen Plaque in Bonbons oder Kaugummis ist beispielsweise aus den Offenlegungsschriften WO 03/013 453 A1, WO 03/013 454 A1 und US-A 2004/258629 bekannt.

Auch wenn durch den verringerten Einsatz von Triclosan die geschmacklichen Probleme sowie Risiken der Verfärbung von Zahnoberflächen minimiert werden, besteht nach wie vor das Bedürfnis, Wirkstoffe oder Wirkstoffkombinationen bereitzustellen, die frei von den genannten Nachteilen sind. Zudem besteht der Wunsch, Zubereitungen bereitzustellen, die ohne den zwingenden Einsatz von Triclosan o.ä. geschmacksneutral hervorragend konserviert und hochwirksam gegen Plaque sind.

Die Aufgabe der vorliegenden Erfindung bestand darin, Zubereitungen zur Mund- und Zahnpflege und -reinigung bereitzustellen, die Mundgeruch wirksam bekämpfen und gegen Gingivitis und Parodontitis wirksam sind. Dabei sollten in einem Aspekt der Erfindung die Nachteile des Einsatzes von Triclosan, Zinnsalzen oder Chlorhexidin vermieden werden können.

Es wurde nun gefunden, daß sich bestimmte synergistische Kombinationen von antibakteriellen Mitteln zur Lösung des vorstehend genannten Aufgabenkomplexes eignen.

Gegenstand der vorliegenden Erfindung ist in einer ersten Ausführungsform ein Mund- und Zahnpflege- und -reinigungsmittel, enthaltend bezogen auf sein Gewicht in einem oral akzeptablen Träger
a) 0,01 bis 2,5 Gew.-% Ethyllauroylarginat
b) 0,00001 bis 2,5 Gew.-% mindestens eines antibakteriellen Mittels aus der Gruppe der
   - Parabene und/oder
   - der Silbersalze und/oder
   - der Kupfersalze und/oder
   - der Zinksalze.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Eine besonders bevorzugte Ausführungsform der vorliegenden Erfindung sind Mundspüllösungen und Mundwasser, die zum Ausspülen der Mundhöhle verwendet werden.

Die erfindungsgemäßen Mittel enthalten als ersten Inhaltsstoff 0,01 bis 2,5 Gew.-% Ethyllauroylarginat (ELA bzw. LAE) 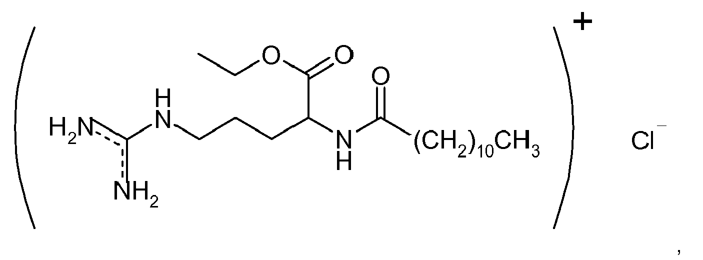 das auch als Ethyl-N^{alpha}-dodecanoyl-L-Arginat Hydrochlorid bezeichnet wird. LAE ist kommerziell erhältlich und gilt als unbedenklich, auch für den Einsatz in Nahrungsmitteln.

Erfindungsgemäß bevorzugte Mund- und Zahnpflege- und -reinigungsmittel enthalten - bezogen auf ihr Gewicht - - 0,05 - 2,25 Gew.-%, vorzugsweise 0,075 - 2 Gew.-%, besonders bevorzugt 0,1 - 1,75 Gew.-%, außerordentlich bevorzugt 0,125 - 1,5 Gew.-% und insbesondere 0,15 bis 0,8 Gew.-% Ethyllauroylarginat.

Als weiteren zwingenden Inhaltsstoff enthalten die erfindungsgemäßen Mund- und Zahnpflege- und -reinigungsmittel mindestens eine weitere antibakterielle Substanz aus der Gruppe der Parabene und/oder der Silbersalze und/oder der Kupfersalze und/oder der Zinksalze.

Parabene sind Ester der para-Hydroxybenzoesäure. Die Ester und deren Natriumsalze sind ebenfalls für den Einsatz in Lebensmitteln zugelassen. Bevorzugte Parabene sind Methylparaben, Ethylparaben, Propylparaben und Butylparaben - Kombinationen von diesen Substanzen mit Ethyllauroylarginat weisen eine gegenüber den Einzelsubstanzen deutlich erhöhte antibakterielle Wirkung auf, reduzieren Plaque deutlich und haben keine geschmacklichen Nachteile.

Besonders bevorzugte erfindungsgemäße Mund- und Zahnpflege- und -reinigungsmittel sind dadurch gekennzeichnet, daß sie - bezogen auf ihr Gewicht - 0,01 - 2 Gew.-%, vorzugsweise 0,025 - 1,75 Gew.-%, besonders bevorzugt 0,04 - 1,5 Gew.-%, außerordentlich bevorzugt 0,075 - 1,25 Gew.-% und insbesondere 0,05 bis 1 Gew.-% Pareben(e) enthalten.

Zusätzlich zu dem/den Paraben(en) oder an dessen/deren Stelle können die erfindungsgemäßen Mittel auch Silberionen enthalten. Diese besitzen in Kombination mit Ethyllauroylarginat ebenfalls synergistische antibakterielle Wirksamkeit und Plaquereduktion und weisen ebenfalls keine geschmacklichen Nachteile auf.

Der Einsatz von Silber als Desinfektionsmittel in der Wundbehandlung, medizinischen Geräten sowie in pharmazeutischen und kosmetischen Zubereitungen ist im Stand der Technik bekannt. Für kosmetische Mittel ist beispielsweise ein Maximalgehalt von 4 % Silbernitrat erlaubt (KosmetikV, Anlage 3 zu § 3, Teil A).

Erfindungsgemäß bevorzugte Mund- und Zahnpflege- und -reinigungsmittel enthalten 0,01 bis 10000 ppm, vorzugsweise 0,05 bis 5000 ppm, weiter bevorzugt 0,1 bis 1000 ppm, noch weiter bevorzugt 1 bis 500 ppm und insbesondere 5 bis 100 ppm Silberionen.

Neben kolloidalem Silber haben sich im Rahmen der vorliegenden Erfindung besonders Silbersalze und darunter insbesondere leicht lösliche Silbersalze als hervorragend geeignet herausgestellt. Besonders bevorzugte erfindungsgemäße Mund- und Zahnpflege- und - reinigungsmittel enthalten mindestens ein Silbersalz ausgewählt aus Silbersulfat, Silbernitrat, Silbercitrat, Silberlactat, Silberacetat, Silbersulfat, Silbermalat, Silbersuccinat, Silbertartrat, Silbermandelat, Silbersalicylat, Silbergluconat, Silberadipat, Silbergalactarat.

Zusätzlich zu dem/den Paraben(en) und/oder dem/den Silbersalz(en) oder an dessen/deren Stelle können die erfindungsgemäßen Mittel auch Kupferionen enthalten. Diese besitzen in Kombination mit Ethyllauroylarginat ebenfalls synergistische antibakterielle Wirksamkeit und Plaquereduktion und weisen ebenfalls keine geschmacklichen Nachteile auf.

Ganz besonders bevorzugte erfindungsgemäße Mund- und Zahnpflege- und -reinigungsmittel enthalten 0,01 bis 10000 ppm, vorzugsweise 0,05 bis 5000 ppm, weiter bevorzugt 0,1 bis 1000 ppm, noch weiter bevorzugt 1 bis 500 ppm und insbesondere 5 bis 100 ppm Kupferionen.

Auch bei den Kupfersalzen haben sich leicht lösliche Kupfersalze als besonders geeignet herausgestellt. Hier sind erfindungsgemäße Mund- und Zahnpflege- und -reinigungsmittel Bevorzugt, die es mindestens ein Kupfersalz ausgewählt aus Kupfersulfat, Kupfernitrat, Kupfercitrat, Kupferlactat, Kupferacetat, Kupfersulfat, Kupfermalat, Kupfersuccinat, Kupfertartrat, Kupfermandelat, Kupfersalicylat, Kupfergluconat, Kupferadipat, Kupfergalactarat enthalten.

Auch Zinksalze können im Rahmen der vorliegenden Erfindung gemeinsam mit Ethyllauroylarginat eingesetzt werden, um eine synergistisch gesteigerte antibakterielle Wirkung und einen im Vergleich zu Triclosan deutlich verbesserten Geschmack zu erzielen.

Bevorzugte erfindungsgemäße Mittel enthalten das bzw. die Zinksalz(e) innerhalb engerer Mengenbereiche. Hier sind erfindungsgemäß bevorzugte Mund- und Zahnpflege- und -reinigungsmittel dadurch gekennzeichnet, daß sie 0,02 bis 2,5 Gew.-%, vorzugsweise 0,03 bis 2,0 Gew.-%, besonders bevorzugt 0,04 bis 1,5 Gew.-%, weiter bevorzugt 0,05 bis 1,0 Gew.-%, noch weiter bevorzugt 0,06 bis 0,5 Gew.-% und insbesondere 0,07 bis 0,25 Gew.-% Zinksalz(e) enthalten. Hierbei gilt - wie immer, wenn in der vorliegenden Schrift keine anderen Angaben gemacht werden - daß sich die Mengenangaben auf das gesamte Mittel, d.h. das fertige Mund und Zahnpflege- und -reinigungsmittel, beziehen.

Erfindungsgemäß können sowohl anorganische als auch organische Salze des Zinks eingesetzt werden. Neben den nicht löslichen anorganischen Zinksalzen, also Salzen, welche eine Löslichkeit unterhalb 100 mg/L (20°C), vorzugsweise unterhalb 10 mg/L (20°C), insbesondere keine Löslichkeit (20°C) aufweisen (Bsp.: Zinkoxid), sind im Rahmen der vorliegenden Anmeldung die löslichen anorganischen Zinksalze, das heißt Salze, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen, bevorzugter Bestandteil erfindungsgemäßer Mittel. Zu den bevorzugten löslichen anorganischen Salzen zählen das Zinkbromid, das Zinkchlorid, das Zinkiodid, das Zinknitrat und das Zinksulfat.
Erfindungsgemäß besonders bevorzugte Mittel enthalten dabei mindestens ein Zinksalz aus der nachstehenden Tabelle:

| Zinksalz | Löslichkeit |
|---|---|
| Zinkacetat-Dihydrat | 430 g/I (20°C) |
| Zinkacetylacetonat | 4 g/I (20°C) |
| Zinkbromid | 820 g/I (25°C) |
| Zinkchlorid | 4320 g/I (25°C) |
| Zinkgluconat | 100 g/I (20°C) |
| Zinkhydroxycarbonat | Fast unlöslich (20°C) |
| Zinkiodid | 4500 g/I (20°C) |
| Zinknitrat Hexahydrat | 1843 g/I (20°C) |
| Zinknitrat-Tetrahydrat | Leicht löslich (20°C) |
| Zinkoxid | Unlöslich |
| Zinkstearat | 0,9 mg/I (20°C) |
| Zinksulfat-Heptahydat | 960 g/I (20°C) |
| Zinksulfat-Monohydrat | ∼350 g/I (20°C) |

Das Spektrum der erfindungsgemäß bevorzugten Zinksalze organischer Säuren, vorzugsweise organischer Carbonsäuren, reicht von Salzen die in Wasser nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinnlaureat, das Zinkoleat, das Zinkoxalat, das Zinktartrat und das Zinkstearat, zu der Gruppe der löslichen organischen Zinksalze gehören beispielsweise das Zinkacetat, das Zinkacetylacetonat, das Zinkbenzoat, das Zinkformiat, das Zinklactat, das Zinkgluconat, das Zinkvalerat sowie das Zinksalz der p-Toluolsulfonsäure.

Es hat sich gezeigt, daß Zinksulfat ein besonders bevorzugt einzusetzendes Zinksalz ist. Zinksulfat bildet mehrere Hydrate. Das Heptahydrat bildet sich aus gesättigter wäßriger Lösung in Form farbloser, glasglänzender, säulenförmiger, rhombischer Kristalle. Oberhalb 39°C erfolgt Umwandlung zu ZnSO₄ · 6H₂O, und bei 70°C liegt nur noch ZnSO₄ · H₂O vor; das letzte H₂O-Molekül entweicht bei 240°C. Interessanterweise steigt die erfindungsgemäße Wirkung bei den Zinksulfaten mit deren Kristallwassergehalt, so daß das Heptahydrat eine besonders bevorzugte Verbindung ist.

Erfindungsgemäß bevorzugte Mund- und Zahnpflege- und -reinigungsmittel enthalten daher Zinksulfat, vorzugsweise Zinksulfat-Heptahydrat.

Die erfindungsgemäßen Mund- und Zahnpflege- und -reinigungsmittel enthalten weitere Inhaltsstoffe. Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.
Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Der Einsatz von Putzkörpern (Abrasiva) ist in den Zahnpasten unter den erfindungsgemäßen Mund- und Zahnpflege- und -reinigungsmitteln ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-Komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 —14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 —250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind solche Zahnpasten unter den erfindungsgemäßen Mund- und Zahnpflege- und -reinigungsmitteln bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ -2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside sowie Fettsäureamidoalkylbetaine.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Ein weiterer Gegenstand der vorliegenden Erfindung ist die Verwendung von Mischungen aus
a) Ethyllauroylarginat
b) mindestens einem antibakteriellen Mittel aus der Gruppe
   - der Parabene und/oder
   - der Silbersalze und/oder
   - der Kupfersalze und/oder
   - der Zinksalze
zur Konservierung kosmetischer Mittel, insbesondere zur Konservierung von Mund- und Zahnpflege- und -reinigungsmitteln.

Bezüglich bevorzugter Ausführungsformen für die erfindungsgemäße Verwendung gilt mutatis mutandis das zu den erfindungsgemäßen Mitteln Gesagte.

### Beispiele:

### Mundspüllösungen (alle Angaben in Gew.-%):

| | **M1** | **M2** | **M3** | **M4** | **M5** | **M6** | **M7** | **M8** |
|---|---|---|---|---|---|---|---|---|
| Sorbitol 70% | 3,0 | 3 | 0 | 3 | 0 | 3 | 0 | 3 |
| Ethyllauroylarginat | 0,4 | 0,5 | 0,4 | 0,5 | 0,6 | 0,7 | 0,7 | 0,8 |
| Methylparaben | 0,2 | | | | | | | |
| Ethyparaben | | 0,2 | | | 0,1 | | | |
| Propylparaben | | | 0,2 | | 0,1 | | | |
| Butylparaben | | | | 0,2 | | | | |
| Silbercitrat | | | | | | 4 ppm Ag⁺ | | |
| Kupfer(II)sulfat | | | | | | | 5 ppm Cu⁺⁺ | |
| Zinksulfat-Heptahydrat | | | | | | | | 0,4 |
| Natriumfluorid | 0,05 | 0,05 | 0,05 | 0,05 | 0,05 | 0,045 | 0,045 | 0,045 |
| Saccharin Natrium | 0,03 | 0,03 | 0,03 | 0,03 | 0,03 | 0,03 | 0,03 | 0,03 |
| Capryl/Caprylyl Glucosid (70% Aktivsubstanz) | 0,86 | 0,86 | 0,86 | 0,86 | 0,86 | 0,86 | 0,86 | 0,86 |
| Aroma | 0,1 | 0,1 | 0,1 | 0,1 | 0,1 | 0,1 | 0,1 | 0,1 |
| Wasser, vollentsalzt | ad 100 | ad 100 | ad 100 | ad 100 | ad 100 | ad 100 | ad 100 | ad 100 |

Es wurden Zahnpasten folgender Zusammensetzung (alle Angaben in Gew.-%)hergestellt:

| | **E1** | **E2** | **E3** | **E4** | **E5** |
|---|---|---|---|---|---|
| Sorbit (70 % DAB) | 60 | 62,5 | 65 | 67,5 | 70,0 |
| Ethanol (96%) | - | - | - | - | 2,0 |
| Fällungskieselsäure : Sident 8 | 10,0 | 10,0 | 10,0 | 10,0 | 12,0 |
| Fällungskieselsäure : Sident 22S | 8,5 | 8,5 | 8,5 | 8,5 | - |
| Poliertonerde P10 feinst | - | 1,0 | - | - | - |
| Dinatriumphosphat, wasserfrei | 0,1 | 0,1 | 0,1 | 0,1 | 0,1 |
| Trinatriumphosphat, wasserfrei | 0,2 | 0,2 | 0,2 | 0,2 | - |
| Na-Monofluorophosphat Na2PO3F | - | - | 1,0 | 1,0 | 1,0 |
| Natriumfluorid | 0,32 | 0,32 | - | - | - |
| Polyethylenglycol (MG : 1500) | 1,5 | 1,5 | 1,5 | 1,5 | 1,5 |
| Titandioxid | 0,5 | 0,5 | 0,5 | 0,5 | - |
| Na-Laurylsulfat | 1,5 | 1,5 | 1,5 | 1,5 | 1,5 |
| Saccharin-Na | 0,1 | 0,1 | 0,1 | 0,1 | 0,1 |
| Xanthan | 0,4 | 0,4 | 0,4 | 0,4 | 0,4 |
| Aroma | 1,2 | 1,2 | 1,2 | 1,2 | 1,2 |
| Ethyllauroylarginat | 0,15 | 0,25 | 0,35 | 0,45 | 0,8 |
| Ethyparaben | 0,3 | | | | |
| Propylparaben | | 0,3 | | | |
| Silbercitrat | | | 4 ppm Ag⁺ | | |
| Kupfer(II)sulfat | | | | 5 ppm Cu⁺⁺ | |
| Zinksulfat-Heptahydrat | | | | | 0,3 |
| Wasser, vollentsalzt | ad 100 | ad 100 | ad 100 | ad 100 | ad 100 |

## Patentansprüche

1. Mund- und Zahnpflege- und -reinigungsmittel, enthaltend bezogen auf sein Gewicht in einem oral akzeptablen Träger
a) 0,01 bis 2,5 Gew.-% Ethyllauroylarginat
b) 0,00001 bis 2,5 Gew.-% mindestens eines antibakteriellen Mittels aus der Gruppe der
- Parabene und/oder
- der Silbersalze und/oder
- der Kupfersalze und/oder
- der Zinksalze.

2. Mund- und Zahnpflege- und -reinigungsmittel nach Anspruch 1, **dadurch gekennzeichnet, daß** es - bezogen auf sein Gewicht - - 0,05 - 2,25 Gew.-%, vorzugsweise 0,075 - 2 Gew.-%, besonders bevorzugt 0,1 - 1,75 Gew.-%, außerordentlich bevorzugt 0,125 - 1,5 Gew.-% und insbesondere 0,15 bis 0,8 Gew.-% Ethyllauroylarginat enthält.

3. Mund- und Zahnpflege- und -reinigungsmittel nach einem der Ansprüche 1 oder 2, **dadurch gekennzeichnet, daß** es - bezogen auf sein Gewicht - 0,01 - 2 Gew.-%, vorzugsweise 0,025 - 1,75 Gew.-%, besonders bevorzugt 0,04 - 1,5 Gew.-%, außerordentlich bevorzugt 0,075 - 1,25 Gew.-% und insbesondere 0,05 bis 1 Gew.-% Pareben(e) enthält.

4. Mund- und Zahnpflege- und -reinigungsmittel nach einem der Ansprüche 1 bis 3, **dadurch gekennzeichnet, daß** es 0,01 bis 10000 ppm, vorzugsweise 0,05 bis 5000 ppm, weiter bevorzugt 0,1 bis 1000 ppm, noch weiter bevorzugt 1 bis 500 ppm und insbesondere 5 bis 100 ppm Silberionen enthält.

5. Mund- und Zahnpflege- und -reinigungsmittel nach einem der Ansprüche 1 bis 4, **dadurch gekennzeichnet, daß** es mindestens ein Silbersalz ausgewählt aus Silbersulfat, Silbernitrat, Silbercitrat, Silberlactat, Silberacetat, Silbersulfat, Silbermalat, Silbersuccinat, Silbertartrat, Silbermandelat, Silbersalicylat, Silbergluconat, Silberadipat, Silbergalactarat enthält.

6. Mund- und Zahnpflege- und -reinigungsmittel nach einem der Ansprüche 1 bis 5, **dadurch gekennzeichnet, daß** es 0,01 bis 10000 ppm, vorzugsweise 0,05 bis 5000 ppm, weiter bevorzugt 0,1 bis 1000 ppm, noch weiter bevorzugt 1 bis 500 ppm und insbesondere 5 bis 100 ppm Kupferionen enthält.

7. Mund- und Zahnpflege- und -reinigungsmittel nach einem der Ansprüche 1 bis 6, **dadurch gekennzeichnet, daß** es mindestens ein Kupfersalz ausgewählt aus Kupfersulfat, Kupfernitrat, Kupfercitrat, Kupferlactat, Kupferacetat, Kupfersulfat, Kupfermalat, Kupfersuccinat, Kupfertartrat, Kupfermandelat, Kupfersalicylat, Kupfergluconat, Kupferadipat, Kupfergalactarat enthält.

8. Mund- und Zahnpflege- und -reinigungsmittel nach einem der Ansprüche1 bis 7, **dadurch gekennzeichnet, daß** es 0,02 bis 2,5 Gew.-%, vorzugsweise 0,03 bis 2,0 Gew.-%, besonders bevorzugt 0,04 bis 1,5 Gew.-%, weiter bevorzugt 0,05 bis 1,0 Gew.-%, noch weiter bevorzugt 0,06 bis 0,5 Gew.-% und insbesondere 0,07 bis 0,25 Gew.-% Zinksalz(e) enthält.

9. Mund- und Zahnpflege- und -reinigungsmittel nach einem der Ansprüche 1 bis 8, **dadurch gekennzeichnet, daß** es Zinksulfat, vorzugsweise Zinksulfat-Heptahydrat, enthält.

10. Verwendung von Mischungen aus
a) Ethyllauroylarginat
b) mindestens einem antibakteriellen Mittel aus der Gruppe
- der Parabene und/oder
- der Silbersalze und/oder
- der Kupfersalze und/oder
- der Zinksalze
zur Konservierung kosmetischer Mittel, insbesondere zur Konservierung von Mund- und Zahnpflege- und -reinigungsmitteln.
